# EUROPEAN PATENT SPECIFICATION

(11) **EP 3 435 986 B1**
(45) Date of publication and mention of the grant of the patent: **27.04.2022**
(21) Application number: 17714789.9
(22) Date of filing: 03.04.2017
(51) Int. Cl.: A61K 9/20, A61K 9/16, A23L 2/39

(54) **NEW TABLETTABLE FORMULATION OF LUTEIN AND/OR ZEAXANTHIN**
NEUE TABLETTIERBARE LUTEIN UND/ODER ZEAXANTHIN ZUBEREITUNG
FORMULATION DE LUTEIN OU/ET ZEAXANTHIN POUVANT ETRE MISES EN COMPRIMES

(30) Priority: 01.04.2016 EP 16163565
(43) Date of publication of application: 06.02.2019
(73) Proprietor: DSM IP Assets B.V., 6411 TE Heerlen (NL)
(72) Inventor: ESTRELLA, Antonio, 4303 Kaiseraugst (CH); FUNDA, Elger, 4303 Kaiseraugst (CH); MISIC, Zdravka, 4303 Kaiseraugst (CH); SCHAEFER, Christian, 4303 Kaiseraugst (CH); BECK, Markus, 4303 Kaiseraugst (CH); SCHLEGEL, Bernd, 4303 Kaiseraugst (CH); URBAN, Kai, 4303 Kaiseraugst (CH)
(74) Representative: Kurt, Manfred
(86) International application number: PCT/EP2017/057812
(87) International publication number: WO 2017/168005

(56) References cited:
- EP-A1- 1 940 249
- WO-A1-94/19411
- WO-A1-2007/009601
- WO-A1-2012/139895
- WO-A1-2012/168189
- WO-A1-2014/154788
- WO-A2-03/022071
- WO-A2-03/066205
- US-A1- 2008 026 124

## Description

The international application WO 2012/139895 relates to a process for the manufacture of a powder containing lutein and further-more to a food composition, especially an instant beverage and effervescent tablets comprising the powder. The international application WO 2007/009601 describes compositions containing finely dispersed carotenoids. The compositions can be used as colorants or additives for food, beverages, animal feeds, cosmetics or drugs. The composition is prepared from a mixture of modified starch with sodium ascorbate, sucrose, maltodextrin and finally added beta-carotene.

The present invention is directed towards a solid formulation comprising a milled carotenoid, at least one hydrocolloid, a glucose syrup, sucrose and at least one water-soluble antioxidant (preferably sodium ascorbate), wherein the carotenoid is selected from the group consisting of lutein and zeaxanthin and any mixture thereof, wherein the hydrocolloid is selected from the group consisting of modified food starches and any mixtures thereof, and wherein the milled carotenoid has the following particle size distribution:
D [3,2] in the range of from 0.6 to 1.5 µm and D [v, 0.5] in the range of from 1.1 to 3.5 µm, all D values as measured by laser diffraction (Malvern Instruments Ltd, Malvern, UK, Mastersizer 3000) according to the Fraunhofer scattering model.

Preferably no other hydrocolloid(s) than modified food starch(es) is/are present in the solid formulation according to the present invention.

Preferably the particle size distribution is measured after the re-dispersed formulation was treated with ultrasound and centrifuged.

Preferably D [3,2] is in the range of from 0.8 to 1.2 µm, more preferably D [3,2] is in the range of from 0.8 to 1.1 µm, as measured by laser diffraction (Malvern Instruments Ltd, Malvern, UK, Mastersizer 3000) according to the Fraunhofer scattering model.

D [v, 0.5] is preferably in the range of from 1.1 to 2.6 µm, more preferably D [v, 0.5] is in the range of from 1.1 to 2.1 µm, as measured by laser diffraction (Malvern Instruments Ltd, Malvern, UK, Mastersizer 3000) according to the Fraunhofer scattering model.

This solid formulation is in form of granules which are used for dietary supplements and pharmaceuticals. These dietary supplements and pharmaceuticals, respectively, are preferably in form of tablets such as e.g. multi-vitamin and/or multi-mineral tablets.

The particle size distribution of the granules is preferably as follows:
D [3,2] in the range of from 200 to 300 µm (preferably in the range of from 230 to 270 µm) and D [v, 0.5] in the range of from 220 to 320 µm (preferably in the range of from 240 to 290 µm), all D values as measured by laser diffraction (Malvern Instruments Ltd, Malvern, UK, Mastersizer 3000) according to the Fraunhofer scattering model.

Preferably the carotenoid is lutein. Lutein plays an important role in eye health. Thus, there is a huge demand for dietary supplements, especially in form of tablets, comprising lutein. The tablets do generally not contain lutein as such, but in form of a formulation, wherein the lutein is embedded in a matrix of a hydrocolloid which protects lutein from degradation and oxidation. Until now such lutein tablets on the market have the disadvantage that they show a high compression loss.

The compression loss is defined as the percentage of carotenoid (especially lutein) which is extractable by an organic solvent out of the formulation, after being tabletted under defined conditions.

The compression loss is a relevant parameter for the shelf life of (pharmaceutical) tablets, i.e. a parameter for the stability of the carotenoid (especially lutein) in the (pharmaceutical) tablets. If the compression loss is smaller, the shelf life of the tablets is longer.

The compression loss is determined by
∘ cautious milling of the tablets to a mix so that the formulation itself is not destroyed by using a mortar;
∘ treating said mix with a suitable solvent (e.g. methylene chloride or petrolether) so that only the carotenoid (especially lutein) which has been pressed out is dissolved;
∘ diluting the solution (solvent + carotenoid, especially lutein) with another solvent (cyclohexane or isopropanol) and
∘ analytical determination of the carotenoid (especially lutein) in the solvent by measuring the absorption of the solution, and
∘ calculation of the percentage of the total amount of the carotenoid (especially lutein) pressed out.

Important: The matrix of the formulation must not be soluble in the organic solvent.

The formulations of the present invention are especially suitable for the preparation of stable tablets/dietary supplements of lutein.

Such tablets show preferably a compression loss of carotenoid (especially lutein) of ≤ 12%, preferably of ≤ 10%, (i.e. a compression loss in the range of from 0 to 10%), i.e. the amount of carotenoid (especially lutein) present at the surface of tablets comprising formulations according to the present invention is ≤ 12 weight-%, preferably ≤ 10 weight-%, based on the total weight of the carotenoid (especially lutein) in the tablet. Carotenoid (especially lutein) present at the surface of such a tablet is a great disadvantage since the carotenoid (especially lutein) is no longer protected against oxidation by the hydrocolloid. Compression losses in the range of from 5 to 12.5 weight-% are quite accepted for most purposes.

### Dietary supplements/Tablets according to the present invention

The formulations according to the present invention are especially suitable for the manufacture of dietary supplements, especially in the form of tablets, with the preferences as given above.

Thus, the present invention is also directed towards tablets comprising a formulation according to the present invention.

Those tablets may further comprise vitamins, mineral salts and/or trace elements.

Examples of vitamins are vitamin E, vitamin C, vitamin K, vitamin B1, vitamin B2, vitamin B6, vitamin B12, vitamin D, biotin, folic acid, niacin, niacin amide, pantothenic acid and/or pantothenate. Instead of these compounds also the corresponding derivatives such as e.g. their esters and salts may be used.

Examples of mineral salts are salts of calcium, phosphor, magnesium, potassium, and chloride.

Examples of trace elements are chrome, cobalt, iron, fluorine (intake as fluoride salts), iodine (intake as iodide salts), copper, manganese, molybdenum, selenium, silicium and zinc.

The solid formulation according to the present invention is now described in more detail.

### Formulations according to the present invention

### Lutein

As starting material most preferably a so-called "lutein cake" as available from Kemin Foods (US) having a lutein content of 50-80 weight-% is used. This lutein cake is obtained by extracting Marigold Flowers. The lutein cake also contains zeaxanthin, whereby the molar ratio of the lutein to zeaxanthin is around 9 : 1.

Lutein obtained from any other natural source or by fermentation or by chemical synthesis may also be used.

In a preferred embodiment of the present invention no other carotenoid except lutein and/or zeaxanthin is present. Excluded of this exception are carotenoids that may already be present in traces in the starting material, e.g. in the "lutein cake".

### Amount of Carotenoid

The amount of the carotenoid is preferably in the range of from 1-30 weight-%, more preferably in the range of from 5-25 weight-%, even more preferably in the range of from 5-17 weight-%, most preferably in the range of from 10-17 weight-%, based on the total weight of the granules.

If the carotenoid is a mixture of lutein and zeaxanthin, their molar ratio is preferably in the range of from 20 : 1 to 2 : 1, more preferably their molar ratio is in the range of from 10 : 1 to 4 : 1.

### Hydrocolloid

The hydrocolloid is selected from the group consisting of modified food starches and any mixture thereof. Preferred are so-called OSA starches.

### "Modified food starch"

A modified food starch is a food starch that has been chemically modified by known methods to have a chemical structure which provides it with a hydrophilic and a lipophilic portion. Preferably the modified food starch has a long hydrocarbon chain as part of its structure (preferably C5-C18).

At least one modified food starch is preferably used to make the solid formulation of this invention, but it is possible to use a mixture of two or more different modified food starches in one solid formulation.

Starches are hydrophilic and therefore do not have emulsifying capacities. However, modified food starches are made from starches substituted by known chemical methods with hydrophobic moieties. For example starch may be treated with cyclic dicarboxylic acid anhydrides such as succinic anhydrides, substituted with a hydrocarbon chain (see O. B. Wurzburg (editor), "Modified Starches: Properties and Uses, CRC Press, Inc. Boca Raton, Florida, 1986, and subsequent editions). A particularly preferred modified food starch of this invention has the following formula (I) 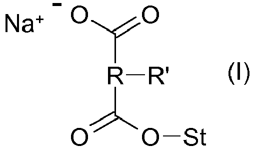 wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree of substitution, i.e. the number of esterified hydroxyl groups to the number of free non-esterified hydroxyl groups usually varies in a range of from 0.1% to 10%, preferably in a range of from 0.5% to 4%, more preferably in a range of from 3% to 4%.

The term "OSA-starch" denotes any starch (from any natural source such as corn, waxy maize, waxy corn, wheat, tapioca and potato or synthesized) that was treated with octenyl succinic anhydride (OSA). The degree of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".

The term "OSA-starches" encompasses also such starches that are commercially available e.g. from National Starch/Ingredion under the tradenames HiCap 100, Capsul (octenylbutanedioate amylodextrin), Capsul HS, Purity Gum 2000, Clear Gum Co03, UNI-PURE, HYLON VII; from National Starch and Roquette Frères, respectively; from CereStar/Cargill under the tradename C*EmCap or from Tate & Lyle.

### Amount of the at least one hydrocolloid (modified starch/OSA starch)

The amount of the modified food starch (preferably the OSA starch) is preferably in the range of from 10 to 50 weight-%, more preferably in the range of from 25 to 45 weight-%, based on the total weight of the formulation.

### Glucose syrup

The glucose syrup can be used as such or in a dried form. Both are commercially available starch hydrolysates, i.e. a mixture of mono-, oligo- and polysaccharides. According to the present invention a dried glucose syrup is preferably used. The preferences given for the dried glucose syrup apply also for the non-dried glucose syrup.

The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

Dried glucose syrup is, as well as non-dried glucose syrup, usually classified by its DE value, which is above 20. According to the present invention preferably a dried glucose syrup is used with a DE in the range of from 20 to 95, more preferably in the range of from 20 to 30, most preferably in the range of from 20 to 23.

In another embodiment of the present invention a mixture of two glucose syrups is used - one having a low DE, preferably a DE ≤ 25, more preferably a DE in the range of from 20 to 25, and the other having a high DE, preferably a DE ≥ 90, more preferably a DE in the range of from 90 to 100.

### Amount of dried glucose syrup

The amount of the dried glucose syrup is in the range of from 0.1 to 40 weight-%, preferably in the range of from 5 to 40 weight-%, more preferably in the range of from 10 to 30 weight-%, most preferably in the range of from 15 to 25 weight-%, based on the total weight of the formulation.

If non-dried glucose syrup is used, it is used in the same amount.

### Sucrose (= saccharose)

In a preferred embodiment of the present invention the weight ratio of the modified food starch to the dried glucose syrup to the sucrose is (1.5-2.5) to (0.5-1.5) to (0.5-1.5), more preferably it is (1.8-2.2) to (0.8-1.2) to (0.8-1.2), most preferably it is 2 to 1 to 1.

### Amount of sucrose

The amount of sucrose is in the range of from 0.1 to 40 weight-%, preferably in the range of from 5 to 40 weight-%, more preferably it is in the range of from 10 to 30 weight-%, most preferably it is in the range of from 15 to 25 weight-%, based on the total weight of the formulation.

In a preferred embodiment of the present invention the amount of the dried glucose syrup and the amount of sucrose is the same in kilograms. In a further preferred embodiment of the present invention the amount of modified food starch in kilograms is the same amount as the total amount of the dried glucose syrup and sucrose in kilograms.

### Water-soluble anti-oxidant

Preferably the water-soluble anti-oxidant is sodium ascorbate, but other water-soluble anti-oxidants being food-grade and thus, suitable for human consumption may also be used.

### Amount of water-soluble anti-oxidant

The amount of waters-soluble anti-oxidant (especially sodium ascorbate) is preferably in the range of from 0.1 to 10 weight-%, more preferably in the range of from 2 to 7 weight-%, most preferably in the range of from 4 to 6 weight-%, based on the total weight of the formulation.

The formulation of the present invention may also contain up to 7 weight-% of water, preferably it contains up to 5 weight-% of water, based on the total weight of the formulation.

In a preferred embodiment of the present invention the amount of the milled carotenoid, the amount of the at least one modified food starch, the amount of the dried glucose syrup, the amount of sucrose and the amount of the water-soluble antioxidant (being preferably sodium ascorbate) sum preferably up to an amount of at least 90 weight-%, preferably of at least 95 weight-%, based on the total weight of the formulation.

In an even more preferred embodiment of the present invention the formulation consists of the milled carotenoid, the at least one modified food starch, the dried glucose syrup, sucrose, the water-soluble antioxidant (being preferably sodium ascorbate) and water.

In a further preferred embodiment of the present invention a lutein cake having a lutein content of 50-80 weight-% is used, whereby the weight ratio of the lutein cake to the matrix consisting of the amount of modified food starch + dried glucose syrup + sucrose is 1 : (4 - 6), preferably 1 : (4.8 - 5.5), more preferably 1 to (5-5.3).

In another preferred embodiment of the present invention a lutein cake having a lutein content of 50-80 weight-% is used, whereby the weight ratio of the lutein cake to the modified food starch is 1 to (1.5-4), preferably 1 to (2-3), more preferably 1 to (2.5-2.7).

In a further preferred embodiment of the present invention a lutein cake having a lutein content of 50-80 weight-% is used, whereby the weight ratio of the lutein cake to the dried glucose syrup is 1 to (0.5-2), preferably 1 to (1.0-1.5), more preferably 1 to (1.25-1.35).

In a further preferred embodiment of the present invention a lutein cake having a lutein content of 50-80 weight-% is used, whereby the weight ratio of the lutein cake to sucrose is 1 to (0.5-2), preferably 1 to (1.0-1.5), more preferably 1 to (1.25-1.35).

Preferably no other compounds are present. Preferably no further hydrocolloids beside modified food starch and no further emulsifiers are present.

Compounds preferably not-being-present are the following ones:
- Hydrolyzed lecithin products, especially those as disclosed on page 5, line 5-19 of WO 2009/071295;
- Gum Acacia; Gum Arabic as e.g. disclosed in WO 2007/009601; also modified as disclosed in WO 2008/110225;
- Gum Ghatti as e.g. described in WO 2009/147158;
- Proteins such as gelatin (fish, swine, bovine gelatin);
- Cellulose derivatives such as e.g. carboxmethylcellulose;
- Plant proteins;
- Milk proteins;
- Ligninsulfonate;
- Conjugates of plant gums and modified food starch, especially those as disclosed in WO 2011/039336;
- Sodium lauryl sulfate and other sodium alkyl sulfates;
- Fat-soluble antioxidants such as e.g. dl-α-tocopherol;
- Isomalt as e.g. used in the process of US 2008/0026124;
- α-zeacarotene;
- β-zeacarotene.

In an especially preferred embodiment of the present invention none of the following compounds is present in the formulation:
- Hydrolyzed lecithin products, especially those as disclosed on page 5, line 5-19 of WO 2009/071295;
- Gum Arabic as e.g. disclosed in WO 2007/009601;
- Fat-soluble antioxidants such as e.g. dl-α-tocopherol;
- Isomalt as e.g. used in the process of US 2008/0026124;
- α-zeacarotene;
- β-zeacarotene.

In a preferred embodiment the solid formulations of the present invention do not contain an oil. The term "oil" does not encompass any lipophilics that may be present in the formulation, because they are part of the lutein cake used as source of lutein.

The term "oil" in the context of the present invention encompasses glycerol and any triglyceride such as vegetable oils or fats like corn oil, sunflower oil, soybean oil, safflower oil, rapeseed oil, peanut oil, palm oil, palm kernel oil, cotton seed oil, olive oil or coconut oil or MCT (middle chain triglycerides) as well as any mixture thereof.

The oils can be from any origin. They can be natural, modified or synthetic. The term "oil" in the context of the present invention thus also encompasses canola oil, sesame oil, hazelnut oil, almond oil, cashew oil, macadamia oil, mongongo nut oil, pracaxi oil, pecan oil, pine nut oil, pistachio oil, sacha Inchi (Plukenetia volubilis) oil or walnut oil.

The present invention also encompasses any combination of any preferred feature of the milled carotenoid as mentioned in this patent application with any preferred feature of the modified food starch, dried glucose syrup, sucrose, water-soluble antioxidant and also their preferred weight ratios and the optional other ingredients of the formulation as mentioned in this specification though not explicitly mentioned.

Thus, any combination of preferred embodiments of the present invention is encompassed by the present invention though not explicitly mentioned.

The **preferred formulation of the present invention** is a solid formulation comprising a milled carotenoid in an amount in the range of from 1 to 30 weight-% (preferably 5 to 25 weight-%), at least one hydrocolloid in an amount in the range of from 10 to 50 weight-% (preferably 25 to 45 weight-%), a dried glucose syrup in an amount in the range of from 0.1 to 40 weight-% (preferably 10 to 30 weight-%), sucrose in an amount in the range of from 0.1 to 40 weight-% (preferably 10 to 30 weight-%), at least one water-soluble antioxidant (preferably sodium ascorbate) in an amount in the range of from 0.1 to 10 weight-% (preferably 2 to 7 weight-%) and water in an amount of from 0 to 7 weight-%, all amounts being based on the weight of the formulation, wherein the carotenoid is selected from the group consisting of lutein and zeaxanthin and any mixture thereof, wherein the hydrocolloid is selected from the group consisting of modified food starches and any mixtures thereof, and wherein the milled carotenoid has the following particle size distribution:
D [3,2] in the range of from 0.6 to 1.5 µm and D [v, 0.5] in the range of from 1.1 to 3.5 µm, all D values as measured by laser diffraction (Malvern Instruments Ltd, Malvern, UK, Mastersizer 3000) according to the Fraunhofer scattering model.

Further preferences of the compounds of the formulation of the present invention (milled carotenoid, modified food starch, glucose syrup, sucrose, water-soluble antioxidant and water) have already been given above.

### Processes for the manufacture of the solid formulations according to the present invention

The process for obtaining the solid formulation according to claim 2 comprises the following steps:
a) providing an aqueous solution of at least one modified food starch, a glucose syrup and sucrose;
b) adding the carotenoid to the solution of step a) hereby obtaining a suspension;
c) milling the suspension of step b) until the following particle size distribution of the milled carotenoid is reached:
   D [3,2] in the range of from 0.6 to 1.5 µm and D [v, 0.5] in the range of from 1.1 to 3.5 µm, both D values as measured by laser diffraction (Malvern Instruments Ltd, Malvern, UK, Mastersizer 3000) according to the Fraunhofer scattering model;
d) spray-granulating the suspension of step c) to obtain the solid formulation according to the present invention;
   whereby a water-soluble antioxidant (preferably being sodium ascorbate) is added during the process.

Optionally a pH adjustment to a pH in the range of from 2.5 to 4.0 (preferably in the range of from 2.9 to 3.5) may be carried out after step c). In a preferred embodiment of the present invention this pH adjustment step is carried out.

By this process a solid formulation in form of granules is obtained. These granules have preferably the following particle size distribution:
D [3,2] in the range of from 200 to 300 µm (preferably in the range of from 230 to 270 µm), D [v, 0.5] in the range of from 220 to 320 µm (preferably in the range of from 240 to 290 µm), both D values as measured by laser diffraction (Malvern Instruments Ltd, Malvern, UK, Mastersizer 3000) according to the Fraunhofer scattering model.

In a preferred embodiment of the present invention step d) is carried out by drying the suspension obtained in step c) by fluid-bed granulation.

### Advantages of the formulation and tablets of the present invention

The solid formulation of the present invention has an excellent flowability so it can be easily added in the manufacturing process of tablets.

The formulation of the present invention shows especially a flowability of at least 100 g/min through an orifice with a diameter of 5 mm, and/or a flowability of at least 250 g/min through an orifice with a diameter of 7 mm and/or a flowability of at least 500 g/min through an orifice with a diameter of 9 mm and/or a flowability of at least 700 g/min through an orifice with a diameter of 10 mm and/or a flowability of at least 2000 g/min through an orifice with a diameter of 15 mm.

In a preferred embodiment the formulation of the present invention shows a flowability in the range of from 100 g/min to 150 g/min through an orifice with a diameter of 5 mm, and/or a flowability in the range of from 250 g/min to 350 g/min through an orifice with a diameter of 7 mm and/or a flowability in the range of from 500 g/min to 750 g/min through an orifice with a diameter of 9 mm and/or a flowability in the range of from 700 g/min to 850 g/min through an orifice with a diameter of 10 mm and/or a flowability in the range of from 2000 g/min to 3000 g/min through an orifice with a diameter of 15 mm.

Multivitamin-multimineral tablets comprising the formulation of the present invention preferably show a hardness as crushing force of at least 70 N when a compression force of 10 kN is applied, and/or a hardness as crushing force of at least 120 N when a compression force of 15 kN is applied, and/or a hardness as crushing force of at least 140 N when a compression force of 17.5 kN is applied, and/or a hardness as crushing force of at least 170 N when a compression force of 20 kN is applied, and/or a hardness as crushing force of at least 200 N when a compression force of 25 kN is applied, and/or a hardness as crushing force of at least 230 N when a compression force of 30 kN is applied, and/or a hardness as crushing force of at least 255 N when a compression force of 35 kN is applied, and/or a hardness as crushing force of at least 285 N when a compression force of 40 kN is applied.

Furthermore, the multivitamin-multimineral tablets comprising the formulation of the present invention show also good tablet properties in terms of friability and disintegration as well as a good chemical stability of the carotenoid and a low compression loss of the carotenoid.

The invention is now further illustrated in the following non-limiting examples.

### Examples

The following abbreviations are used: RH = room humidity.

### Measurement of particle size

All particle sizes of the solid particles of the present invention are determined by laser diffraction technique using a "Mastersizer 3000" of Malvern Instruments Ltd., UK. Further information on this particle size characterization method can e.g. be found in "Basic principles of particle size analytics", Dr. Alan Rawle, Malvern Instruments Limited, Enigma Business Part, Grovewood Road, Malvern, Worcestershire, WR14 1XZ, UK and the "Manual of Malvern particle size analyzer". Particular reference is made to the user manual number MAN 0096, Issue 1.0, Nov. 1994.

### Analytical equipment that was used for powder and tablet characterization

Powder characteristics included the analyses of powder density (bulk and tapped) and flowability. Bulk (ρbulk) and tapped densities (ρtapped) are determined with an Engelsmann JEL powder tap density tester (J. Engelsmann AG, Ludwigshafen, Germany) according to EP <2.9.34> and USP <616> via the measurement in a graduated cylinder. The powder flowability is determined with a Pharmatest PTG-S4 automated powder characterization instrument (Pharma Test Apparatebau AG, Hainburg, Germany). Mass flow rate (g/min) is determined via the method of flow through an orifice. Flow rate is interpreted as the time needed for a specified amount of powder (100 g) to flow through an orifice with different diameters. A free flowing powder should be able to flow through the whole set of diameters 5, 7, 9, 10, and 15 mm. The plot of flow rate vs. orifice diameter is referred as flow curve. Three parallel measurements are performed to determine the flow rate.

Following tableting, the characterization of compressed tablets including tablet hardness, friability, disintegration, and content uniformity is done. The breaking strengths of tablets (hardness) are measured as described in USP <1217> and EP <2.9.8.> with a Krämer UTS4 1 tester (Kraemer Elektronik GmbH, Darmstadt, Germany). The force needed to break a tablet axially is measured. Presented are always average values of 10 measurements.

Friability, that is closely related to tablet hardness, refers to the extent of weight loss during mechanical abrasion. A maximum loss of no more than 1% of the initial tablet weight is considered acceptable (USP <1216>, EP < 2.9.7.>). 10 tablets are tested in an AE-1 Friabilator (Charles Ischi AG Pharma Prüftechnik, Zuchwill, Switzerland) at a rotation speed of 25 rpm for 4 minutes. The weight loss of the tablets is recorded.

Tablet disintegration is characterized according to USP<701, 2040> by using a DISI-1 disintegration tester (Charles Ischi PG Pharma Prüftechnik, Zuchwill, Switzerland) in 900 mL demineralized water at 37°C. Six parallel measurements are carried out. Upper limit of disintegration time is 30 min for uncoated tablets (USP <2040>).

Content uniformity is evaluated via the standard deviation RSD (%) calculated out of 10 individual determinations per series.

Stability of prototypes in tablet application has been monitored at 25° C/60% RH (long-term study, up to 36 months), 30° C/65% RH (intermediate stability, 12 months) and at 40° C/75% RH (accelerated/stress test, 6 months). The tablets are stored in sealed polyethylene (PE) boxes, and analyzed at pre-determined time-points.

### Measurement of the particle size distribution

The particle size distribution was measured after the re-dispersed formulation of example 1, 2 and 3, respectively, was treated with ultrasound and centrifuged.

### Example 1: Manufacture of a solid formulation of lutein and zeaxanthin according to the present invention

150 kg of OSA-Starch, 75 kg of dried glucose syrup and 75 kg of sucrose are dissolved in 440 l of preheated water at 72°C for at least 30 minutes (matrix). 60 kg of FloraGlo lutein crystals (as available from Kemin Foods, Des Moines, US) are then added to the matrix under stirring at a temperature between 36°C and 20°C. After pH adjustment of the resulting suspension to a pH of 3.5 the resulting pH-adjusted suspension is added to the milling beads (diameter of 0.3 mm) and milling is carried out in several passages. To the resulting suspension 18 kg of sodium ascorbate are added. Then water is added and spray dry granulation started. 275 kg of the solid formulation are obtained.

### Example 2: Manufacture of a solid formulation of lutein and zeaxanthin according to the present invention

160 kg of OSA-Starch, 80 kg of dried glucose syrup and 80 kg of sucrose are dissolved in 480 l of preheated water at 72°C for at least 30 minutes (matrix). 60 kg of FloraGlo lutein crystals (as available from Kemin Foods, Des Moines, US) are then added to the matrix under stirring at a temperature between 36°C and 29°C. After pH adjustment of the resulting suspension to a pH of 2.9 the resulting pH-adjusted suspension is added to the milling beads (diameter of 0.3 mm) and milling is carried out in several passages. To the resulting suspension 20 kg of sodium ascorbate are added. Then water is added and spray dry granulation started. 305 kg of the solid formulation are obtained.

### Example 3: Manufacture of a solid formulation of lutein and zeaxanthin according to the present invention

160 kg of OSA-Starch, 80 kg of dried glucose syrup and 80 kg of sucrose are dissolved in 480 l of preheated water at 72°C for at least 30 minutes (matrix). 60 kg of FloraGlo lutein crystals (as available from Kemin Foods, Des Moines, US) are then added to the matrix under stirring at a temperature between 39°C and 21 °C. After pH adjustment of the resulting suspension to a pH of 3.03 the resulting pH-adjusted suspension is added to the milling beads (diameter of 0.3 mm) and milling is carried out in several passages. To the resulting suspension 18 kg of sodium ascorbate are added. Then water is added and spray dry granulation started. 330 kg of the solid formulation are obtained.

### Example 4: Comparison example

As comparison example the commercially available Lutemax2020^{™} (Lutein & Zeaxanthin Vegetarian Multifunctional beadlets 5%) (OmniActive Health Technologies, India) is used. It is a standardized extract of dried flowers of Marigold (Tagetes species) in the form of purified Free Lutein concentrate encapsulated in a matrix of hydrophilic carriers, cellulose derivatives and natural anti-oxidants.

### Flowability

All three formulations manufactured according to examples 1-3 showed an excellent flowability (see table 1 below).

**Table 1:**

| **Flow [g/min] through an orifice with a diameter of** | **Example 1-Flow [g/min]** | **Example 2-Flow [g/min]** | **Example 3-Flow [g/min]** |
|---|---|---|---|
| **15 mm** | 2308 | 2308 | 2368 |
| **10 mm** | 789 | 789 | 796 |
| **9 mm** | 571 | 657 | 672 |
| **7 mm** | 300 | 299 | 305 |
| **5 mm** | 126 | 126 | 124 |

### Density

The bulk density and the tapped density of all three examples is high as can be seen in the following table 2.

**Table 2:**

| | **Example 1** | **Example 2** | **Example 3** |
|---|---|---|---|
| **Bulk density** | 0.71 g/cm³ | 0.71 g/cm³ | 0.71 g/cm³ |
| **Tapped density** | 0.79 g/cm³ | 0.79 g/cm³ | 0.79 g/cm³ |

### Example 5: Tableting process and equipment

Tablet mixtures are prepared by mixing and sieving (1 mm diameter of the sieve) the ingredients as given in table 3 and 4, respectively, in a Turbula mixer (Willy A. Bachofen Maschinenfabrik) at 23 rpm for 15 min followed by mixing in a drum (7.5 l) for 15 min at 20 rpm (rotations per minute).

**Table 3: Vitamin premix (dose in mg per tablet)**

| **Ingredient** | **As commercially available form** | **Amount of ingredient in form** | **Dose [mg]** | **Total amount in [g]** | **Amount in [%]** |
|---|---|---|---|---|---|
| Beta carotene | Beta Tab 20% S | 20% | 7.50 | 90.00 | 3.89 |
| Vitamin A | Vitamin A Acetate 500 | 500'000 IU | 8.10 | 97.20 | 4.21 |
| Vitamin D3 | Vitamin D3 100 SD/S | 100'000 IU | 5.40 | 64.80 | 2.80 |
| Vitamin C | Ascorbic Acid 90% granulation | 90% | 70.00 | 840.00 | 36.35 |
| Vitamin E | Vitamin E 75 HP | 750 IU | 42.00 | 504.00 | 21.81 |
| Vitamin B1 | Thiamine mononitrate | 100% | 2.03 | 24.35 | 1.05 |
| Vitamin B2 | Riboflavin TG | 100% | 1.87 | 22.44 | 0.97 |
| Vitamin B3 | Niacinamide | 100% | 21.00 | 252.00 | 10.90 |
| Vitamin B5 | Calcium D-Panthothenate | 100% | 14.72 | 176.58 | 7.64 |
| Vitamin B6 | Pyridoxine Hydrochloride | 100% | 2.68 | 32.21 | 1.39 |
| Vitamin B8 | Biotin 1% on Potato maltodextrin | 1% | 3.75 | 45.00 | 1.95 |
| Vitamin B9 | Folic acid 10% on Potato maltodextrin | 10% | 5.00 | 60.00 | 2.60 |
| Vitamin B12 | Vitamin B12 0.1% WS N | 0.1% | 7.80 | 93.60 | 4.05 |
| Vitamin K1 | Vitamin K1 5% SD | 5% | 0.75 | 9.00 | 0.39 |
| | | | Σ = 192.60 | Σ =2311.18 | Σ = 100.00 |

**Table 4: Mineral premix (dose in mg per tablet)**

| **Ingredient** | **As commercially available form** | **Amount of ingredient in form [%]** | **Dose [mg]** | **Total amount in [g]** | **Amount in [%]** |
|---|---|---|---|---|---|
| iron | Ferrous fumarate | 31.52 | 57.11 | 611.04 | 28.60 |
| copper | Copper (II) sulphate anhydrous | 39.82 | 5.02 | 53.74 | 2.51 |
| manganese | Manganese sulphate monohydrate | 32.50 | 6.15 | 65.85 | 3.08 |
| zinc | Zinc sulphate monohydrate | 36.40 | 41.21 | 440.93 | 20.63 |
| iodine | Potassium iodide 10% on Potato maltodextrin | 7.43 | 2.02 | 21.60 | 1.01 |
| chrome | Chromic chloride hexahydrate | 19.52 | 0.31 | 3.29 | 0.15 |
| selenium | Sodium selenite 1% on corn maltodextrin | 0.44 | 11.36 | 121.59 | 5.69 |
| molybdene | Sodium molybdate dihydrate | 39.60 | 0.19 | 2.03 | 0.09 |
| Potassium chloride | Potassium chloride | 52.40 | 76.34 | 816.79 | 38.22 |
| | | | Σ = 199.71 | Σ = 2136.86 | Σ = 100.00 |

Tablets are compressed with a Korsch XL 100 rotary tablet press (Korsch AG, Berlin, Germany). During tableting, the tablet mixtures are compressed with different compression forces (10 - 40 kN) and the breaking force of the tablets is measured. Tablet hardness vs. compression force is plotted to construct the compression profiles. Based on the obtained compression profiles, the optimal compression force was determined (20 kN) and the tablets are produced with the composition as given in the following table 5.

**Table 5: Tablet composition of the multivitamin-multimineral tablets 1-3 and comparison table 4 comprising either of the formulations according to example 1, 2 or 3 or the formulation according to comparison example 4**

| **Ingredient** | **Tablet 1 with formulation according to example 1** | **Tablet 2 with formulation according to example 2** | **Tablet 3 with formulation according to example 3** | **Comparison tablet 4 with formulation according to comparison example 4** |
|---|---|---|---|---|
| **Lutein/zeaxanthin** | In form of formulation according to example 1: 60.00 mg | In form of formulation according to example 2: 60.00 mg | In form of formulation according to example 3: 60.00 mg | In form of formulation according to example 4: 120.00 mg |
| **Vitamin premix according to table 6** | 192.60 mg | 192.60 mg | 192.60 mg | 192.60 mg |
| **mineral premix according to table 7** | 199.71 mg | 199.71 mg | 199.71 mg | 199.71 mg |
| **Magnesium as magnesium oxide tornita** | 166.67 mg | 166.67 mg | 166.67 mg | 166.67 mg |
| **Calcium and phosphorous as calcium phosphate anhydrous (Emcompress)** | 594.90 mg | 594.90 mg | 594.90 mg | 594.90 mg |
| **Crospovidone NF as Polyplasdone XL** | 7.00 mg | 7.00 mg | 7.00 mg | 7.00 mg |
| **Silicium as Aerosil 200** | 4.28 mg | 4.28 mg | 4.28 mg | 4.28 mg |
| **Stearic Acid powder** | 4.00 mg | 4.00 mg | 4.00 mg | 4.00 mg |
| **Magnesium stearate** | 4.00 mg | 4.00 mg | 4.00 mg | 4.00 mg |
| **Cellulose, microcrystalline as Avicel PH 102** | 311.85 mg | 311.85 mg | 311.85 mg | 311.85 mg |
| **ad** | 1500.00 mg | 1500.00 mg | 1500.00 mg | 1500.00 mg |

### Compression profile (see table 6)

The compression profiles of all three tablets 1-3 are similar to each other. The comparison example exhibited lower compressibility what was reflected in higher compression force needed for tableting - at least 25 kN for the comparison tablet 4 versus 20 kN for the tablets 1-3 according to the invention.

**Table 6:**

| **Compression force Fₚᵣₑₛₛ [kN]** | **Tablet 1 with formulation according to example 1** - **average of hardness [N]** | **Tablet 2 with formulation according to example 2-average of hardness [N]** | **Tablet 3 with formulation according to example 3** - **average of hardness [N]** |
|---|---|---|---|
| 10.0 | 78 | 75 | 79 |
| 15.0 | 128 | 122 | 123 |
| 17.5 | 144 | 148 | 149 |
| 20.0 | 178 | 175 | 175 |
| 25.0 | 207 | 207 | 208 |
| 30.0 | 246 | 239 | 242 |
| 35.0 | 266 | 269 | 261 |
| 40.0 | 299 | 290 | 298 |

### Tablet characteristics

All three tablets 1-3 according to the present invention show satisfactory tablet properties in terms of hardness, friability and disintegration (see table 7). That means that multivitamin-multimineral tablets with the formulations according to the present invention show a hardness as crushing force of at least 160 N when a compression force of 20 kN - 25 kN is applied.

The comparison tablet 4 exhibits a longer disintegration time compared to the tablets 1-3 according to the present invention. The content uniformity within all produced tablets according to the present invention is satisfactory (RSD < 5%).

**Table 7**

| | **Tablet 1** | **Tablet 2** | **Tablet 3** | **Comparison tablet 4** |
|---|---|---|---|---|
| **Tablet press** | Korsch XL 100 | Korsch XL 100 | Korsch XL 100 | Korsch XL 100 |
| **Punch** | 22 × 9 mm | 22 × 9 mm | 22 × 9 mm | 22 × 9 mm |
| **kN** | 20 | 20 | 20 | 25 |
| **Weight of cores [mg]** | 1509.3 (1501.0 - 1515.5) | 1505.7 (1499.9 - 1512.0) | 1507.1 (1500.4 - 1514.1) | 1499.1 (1490.7 - 1507.3) |
| **Hardness [N]** | 175 (166 - 180) | 172 (166 - 182) | 170 (162 - 179) | 166 (156 - 176) |
| **Friability [%]** | 0.16 | 0.13 | 0.12 | 0.09 |
| **Disintegration of cores in deionized water at 37°C** | 1 min 26 sec (1 min 20 sec - 1 min 32 sec) | 1 min 28 sec (1 min 22 sec - 1 min 40 sec) | 1 min 29 sec (1 min 16 sec - 1 min 44 sec) | 5 min 58 sec (4 min 50 sec - 7 min 36 sec) |

### Stability trials with the tablets comprising the formulations manufactured according to example 1, 2 and 3, respectively

The lutein and zeaxanthin contents of the three tablets comprising the formulations according to the examples 1-3 and the lutein and zeaxanthin contents of the tablet comprising the form of the comparison example 4 have been measured at the following conditions:
25°C at 60% RH, 30°C at 65% RH and 40°C at 75% RH.

The results are shown in the following tables 8-10.

**Table 8: 25°C at 60% RH**

| **Sample** | **Lutein content in % of initial amount** | | | | **Zeaxanthin content in % of initial amount** | | | |
|---|---|---|---|---|---|---|---|---|
| **Time** | **0** | **1 month** | **2 months** | **3 months** | **0** | **1 month** | **2 months** | **3 months** |
| Tablet 1 | 98.5 | 85.5 | 80.3 | 80.3 | 100.5 | 86.5 | 81.4 | 84.8 |
| Tablet 2 | 97.9 | 88.6 | 85.7 | 83.5 | 97.6 | 87.3 | 85.7 | 87.3 |
| Tablet 3 | 96.7 | 84.9 | 83.3 | 80.8 | 96.6 | 84.1 | 84.5 | 84.1 |
| Comparison tablet 4 | 99.7 | 75.9 | 68.3 | 55.5 | 100.3 | 77.3 | 71.3 | 61.5 |

**Table 9: 30°C at 65% RH**

| **Sample** | **Lutein content in % of initial amount** | | | | **Zeaxanthin content in % of initial amount** | | | |
|---|---|---|---|---|---|---|---|---|
| **Time** | **0** | **1 month** | **2 months** | **3 months** | **0** | **1 month** | **2 months** | **3 months** |
| Tablet 1 | 98.5 | 82.7 | 80.6 | 78.5 | 100.5 | 83.1 | 81.4 | 81.4 |
| Tablet 2 | 97.9 | 86.2 | 83.2 | 83 | 97.6 | 84.1 | 83.7 | 87.3 |
| Tablet 3 | 96.7 | 84.5 | 81 | 79.7 | 96.6 | 82.5 | 81.9 | 84.1 |
| Comparison tablet 4 | 99.7 | 73.9 | 60.3 | 55.4 | 100.3 | 76.1 | 64.1 | 60.4 |

**Table 10: 40°C at 75% RH**

| **Sample** | **Lutein content in % of initial amount** | | | | **Zeaxanthin content in % of initial amount** | | | |
|---|---|---|---|---|---|---|---|---|
| **Time** | **0** | **1 month** | **2 months** | **3 months** | **0** | **1 month** | **2 months** | **3 months** |
| Tablet 1 | 98.5 | 84.8 | 81 | 76.8 | 100.5 | 84.8 | 81.4 | 81.4 |
| Tablet 2 | 97.9 | 86.6 | 84.4 | 81.3 | 97.6 | 85.7 | 85.1 | 85.7 |
| Tablet 3 | 96.7 | 83.3 | 80.4 | 79.1 | 96.6 | 82.5 | 82.8 | 82.5 |
| Comparison tablet 4 | 99.7 | 56.7 | 45.9 | 25.6 | 100.3 | 62.8 | 52 | 33.2 |

## Claims

1. A solid formulation comprising a milled carotenoid, at least one hydrocolloid, a glucose syrup, sucrose and a water-soluble antioxidant,
wherein the carotenoid is selected from the group consisting of lutein and zeaxanthin and any mixture thereof,
wherein the hydrocolloid is selected from the group consisting of modified food starches and any mixture thereof,
and wherein the milled carotenoid has the following particle size distribution: D [3,2] in the range of from 0.6 to 1.5 µm, and D [v, 0.5] in the range of from 1.1 to 3.5 µm, all D values as measured by laser diffraction according to the Fraunhofer scattering model.

2. The solid formulation according to claim 1, wherein the solid formulation is in the form of granules.

3. The solid formulation according to claim 2, wherein the granules have the following particle size distribution:
D [3,2] in the range of from 200 to 300 µm (preferably in the range of from 230 to 270 µm) and D [v, 0.5] in the range of from 220 to 320 µm (preferably in the range of from 240 to 290 µm), all D values as measured by laser diffraction according to the Fraunhofer scattering model.

4. The solid formulation according to any one or more of the preceding claims, wherein the amount of sucrose is in the range of from 0.1 to 40 weight-%, preferably in the range of from 10 to 30 weight-%, more preferably in the range of from 15 to 25 weight-%, based on the total weight of the formulation.

5. The solid formulation according to any one or more of the preceding claims, wherein the amount of carotenoid is in the range of from 1 to 30 weight-%, preferably in the range of from 5 to 25 weight-%, more preferably in the range of from 5 to 17 weight-%, most preferably in the range of from 10 to 17 weight-%, based on the total weight of the formulation.

6. The solid formulation according to any one or more of the preceding claims, wherein the carotenoid is a mixture of lutein and zeaxanthin, and wherein the molar ratio of lutein to zeaxanthin is in the range of from 20 : 1 to 2 : 1, preferably the molar ratio is in the range of from 10 : 1 to 4 : 1.

7. The solid formulation according to any one or more of the preceding claims, wherein the amount of the modified food starch is in the range of from 10 to 50 weight-%, preferably in the range of from 25 to 45 weight-%, based on the total weight of the formulation.

8. The solid formulation according to any one or more of the preceding claims, wherein the amount of glucose syrup is in the range of from 0.1 to 40 weight-%, preferably in the range of from 10 to 30 weight-%, more preferably in the range of from 15 to 25 weight-%, based on the total weight of the formulation.

9. The solid formulation according to any one or more of the preceding claims, wherein the amount of the water-soluble antioxidant (preferably being sodium ascorbate) is in the range of from 0.1 to 10 weight-%, preferably in the range of from 2 to 7 weight-%, more preferably in the range of from 4 to 6 weight-%, based on the total weight of the formulation.

10. The solid formulation according to any one or more of the preceding claims, wherein the amount of the milled carotenoid, the amount of the at least one modified food starch, the amount of the glucose syrup, the amount of sucrose and the amount of the water-soluble antioxidant (preferably being sodium ascorbate) sum up to an amount of at least 90 weight-%, preferably of at least 95 weight-%, based on the total weight of the formulation.

11. The solid formulation according to any one or more of the preceding claims, wherein the formulation does not contain any oil.

12. The solid formulation according to any one or more of the preceding claims, wherein the formulation does not comprise any of the following compounds: hydrolyzed lecithin products, Gum Arabic, fat-soluble antioxidants, isomalt, α-zeacarotene and β-zeacarotene.

13. A dietary supplement comprising a solid formulation according to any one or more of the preceding claims.

14. The dietary supplement according to claim 13 further comprising vitamins, mineral salts and/or trace elements.

15. The dietary supplement according to claim 13 and/or 14, wherein the dietary supplement is in the form of a tablet.

16. The tablet according to claim 15, wherein the tablet has a compression loss of less than 12% of carotenoid, based on the total amount of carotenoid.

17. The tablet according to claim 15 and/or 16, wherein the tablet is a multivitamin-multimineral tablet showing a hardness as crushing force of at least 70 N when a compression force of 10 kN is applied.

18. Use of a solid formulation according to one or more of claims 1-12 as additive for dietary supplements, especially for dietary supplements in form of tablets.

19. A process for obtaining the solid formulation according to claim 2, comprising the following steps:
a) providing an aqueous solution of at least one modified food starch, a glucose syrup and sucrose;
b) adding the carotenoid to the solution of step a) hereby obtaining a suspension;
c) milling the suspension of step b) until the particle size distribution of the milled carotenoid according to claim 1 is reached;
d) spray-granulating the suspension of step c) to obtain the solid formulation according to any one or more of claims 2-12;
whereby a water-soluble antioxidant (being preferably sodium ascorbate) is added during the process.

## Patentansprüche

1. Feste Formulierung, umfassend ein gemahlenes Carotinoid, mindestens ein Hydrokolloid, einen Glucosesirup, Saccharose und ein wasserlösliches Antioxidans,
wobei das Carotinoid aus der Gruppe bestehend aus Lutein und Zeaxanthin und einer Mischung davon ausgewählt ist,
wobei das Hydrokolloid aus der Gruppe bestehend aus modifizierten Lebensmittelstärken und einer Mischung davon ausgewählt ist
und wobei das gemahlene Carotinoid die folgende Partikelgrößenverteilung aufweist:
D [3,2] im Bereich von 0,6 bis 1,5 µm und D [v, 0,5] im Bereich von 1,1 bis 3,5 µm, alle D-Werte wie gemessen durch Laserbeugung gemäß dem Fraunhofer-Streumodell.

2. Feste Formulierung nach Anspruch 1, wobei die feste Formulierung in Form von Granulat vorliegt.

3. Feste Formulierung nach Anspruch 2, wobei das Granulat die folgende Partikelgrößenverteilung aufweist:
D [3,2] im Bereich von 200 bis 300 µm (vorzugsweise im Bereich von 230 bis 270 µm) und D [v, 0,5] im Bereich von 220 bis 320 µm (vorzugsweise im Bereich von 240 bis 290 µm), alle D-Werte wie gemessen durch Laserbeugung gemäß dem Fraunhofer-Streumodell.

4. Feste Formulierung nach einem oder mehreren der vorhergehenden Ansprüche, wobei die Menge an Saccharose im Bereich von 0,1 bis 40 Gew.-%, vorzugsweise im Bereich von 10 bis 30 Gew.-%, weiter bevorzugt im Bereich von 15 bis 25 Gew.-%, bezogen auf das Gesamtgewicht der Formulierung, liegt.

5. Feste Formulierung nach einem oder mehreren der vorhergehenden Ansprüche, wobei die Menge an Carotinoid im Bereich von 1 bis 30 Gew.-%, vorzugsweise im Bereich von 5 bis 25 Gew.-%, weiter bevorzugt im Bereich von 5 bis 17 Gew.-%, ganz besonders bevorzugt im Bereich von 10 bis 17 Gew.-%, bezogen auf das Gesamtgewicht der Formulierung, liegt.

6. Feste Formulierung nach einem oder mehreren der vorhergehenden Ansprüche, wobei es sich bei dem Carotinoid um eine Mischung von Lutein und Zeaxanthin handelt und wobei das Molverhältnis von Lutein zu Zeaxanthin im Bereich von 20:1 bis 2:1 liegt und das Molverhältnis vorzugsweise im Bereich von 10:1 bis 4:1 liegt.

7. Feste Formulierung nach einem oder mehreren der vorhergehenden Ansprüche, wobei die Menge der modifizierten Stärke im Bereich von 10 bis 50 Gew.-%, vorzugsweise im Bereich von 25 bis 45 Gew.-%, bezogen auf das Gesamtgewicht der Formulierung, liegt.

8. Feste Formulierung nach einem oder mehreren der vorhergehenden Ansprüche, wobei die Menge an Glucosesirup im Bereich von 0,1 bis 40 Gew.-%, vorzugsweise im Bereich von 10 bis 30 Gew.-%, weiter bevorzugt im Bereich von 15 bis 25 Gew.-%, bezogen auf das Gesamtgewicht der Formulierung, liegt.

9. Feste Formulierung nach einem oder mehreren der vorhergehenden Ansprüche, wobei die Menge des wasserlöslichen Antioxidans (bei dem es sich vorzugsweise um Natriumascorbat handelt) im Bereich von 0,1 bis 10 Gew.-%, vorzugsweise im Bereich von 2 bis 7 Gew.-%, weiter bevorzugt im Bereich von 4 bis 6 Gew.-%, bezogen auf das Gesamtgewicht der Formulierung, liegt.

10. Feste Formulierung nach einem oder mehreren der vorhergehenden Ansprüche, wobei sich die Menge des gemahlenen Carotinoids, die Menge der mindestens einen modifizierten Lebensmittelstärke, die Menge des Glucosesirups, die Menge der Saccharose und die Menge des wasserlöslichen Antioxidans (bei dem es sich vorzugsweise um Natriumascorbat handelt) zu einer Menge von mindestens 90 Gew.-%, vorzugsweise von mindestens 95 Gew.-%, bezogen auf das Gesamtgewicht der Formulierung, summieren.

11. Feste Formulierung nach einem oder mehreren der vorhergehenden Ansprüche, wobei die Formulierung kein Öl enthält.

12. Feste Formulierung nach einem oder mehreren der vorhergehenden Ansprüche, wobei die Formulierung keine der folgenden Verbindungen umfasst: hydrolysierte Lecithinprodukte, Gummi arabicum, fettlösliche Antioxidantien, Isomalt, α-Zeacarotin und β-Zeacarotin.

13. Nahrungsergänzungsmittel, umfassend eine feste Formulierung nach einem oder mehreren der vorhergehenden Ansprüche.

14. Nahrungsergänzungsmittel nach Anspruch 13, ferner umfassend Vitamine, Mineralsalze und/oder Spurenelemente.

15. Nahrungsergänzungsmittel nach Anspruch 13 und/oder 14, wobei das Nahrungsergänzungsmittel in Form einer Tablette vorliegt.

16. Tablette nach Anspruch 15, wobei die Tablette einen Kompressionsverlust von weniger als 12 % Carotinoid, bezogen auf die Gesamtmenge an Carotinoid, aufweist.

17. Tablette nach Anspruch 15 und/oder 16, wobei es sich bei der Tablette um eine Multivitamin-Multimineral-Tablette mit einer Härte als Bruchkraft von mindestens 70 N bei Anwendung einer Kompressionskraft von 10 kN handelt.

18. Verwendung einer festen Formulierung nach einem der Ansprüche 1-12 als Additiv für Nahrungsergänzungsmittel, insbesondere für Nahrungsergänzungsmittel in Form von Tabletten.

19. Verfahren zum Erhalt der festen Zusammensetzung nach Anspruch 2, das folgende Schritte umfasst:
a) Bereitstellen einer wässrigen Lösung von mindestens einer modifizierten Lebensmittelstärke, einem Glucosesirup und Saccharose;
b) Zugeben des Carotinoids zu der Lösung aus Schritt a), wodurch eine Suspension erhalten wird;
c) Mahlen der Suspension aus Schritt b), bis die Partikelgrößenverteilung des gemahlenen Carotinoids gemäß Anspruch 1 erreicht ist;
d) Sprühgranulieren der Suspension aus Schritt c), wodurch die feste Formulierung nach einem oder mehreren der Ansprüche 2-12 erhalten wird;
wobei während des Verfahrens ein wasserlösliches Antioxidans (bei dem es sich vorzugsweise um Natriumascorbat handelt) zugegeben wird.

## Revendications

1. Formulation solide comprenant un caroténoïde broyé, au moins un hydrocolloïde, un sirop de glucose, du saccharose et un antioxydant soluble dans l'eau,
le caroténoïde étant choisi dans le groupe constitué par la lutéine et la zéaxanthine et un quelconque mélange correspondant,
l'hydrocolloïde étant choisi dans le groupe constitué par des amidons alimentaires modifiés et un quelconque mélange correspondant,
et le caroténoïde broyé possédant la distribution de taille de particule suivante : D [3,2] dans la plage allant de 0,6 à 1,5 µm, et D [v, 0,5] dans la plage allant de 1,1 à 3,5 µm, toutes les valeurs D étant telles que mesurées par diffraction laser selon le modèle de diffusion de Fraunhofer.

2. Formulation solide selon la revendication 1, la formulation solide étant sous la forme de granules.

3. Formulation solide selon la revendication 2, les granules possédant la distribution de taille de particule suivante :
D [3,2] dans la plage allant de 200 à 300 µm (préférablement dans la plage allant de 230 à 270 µm) et D [v, 0,5] dans la plage allant de 220 à 320 µm (préférablement dans la plage allant de 240 à 290 µm), toutes les valeurs D étant telles que mesurées par diffraction laser selon le modèle de diffusion de Fraunhofer.

4. Formulation solide selon l'une quelconque ou plusieurs des revendications précédentes, la quantité de saccharose étant dans la plage allant de 0,1 à 40 % en poids, préférablement dans la plage allant de 10 à 30 % en poids, plus préférablement dans la plage allant de 15 à 25 % en poids, sur la base du poids total de la formulation.

5. Formulation solide selon l'une quelconque ou plusieurs des revendications précédentes, la quantité de caroténoïde étant dans la plage allant de 1 à 30 % en poids, préférablement dans la plage allant de 5 à 25 % en poids, plus préférablement dans la plage allant de 5 à 17 % en poids, le plus préférablement dans la plage allant de 10 à 17 % en poids, sur la base du poids total de la formulation.

6. Formulation solide selon l'une quelconque ou plusieurs des revendications précédentes, le caroténoïde étant un mélange de lutéine et de zéaxanthine, et le rapport molaire de lutéine sur zéaxanthine étant dans la plage allant de 20 : 1 à 2 : 1, préférablement le rapport molaire étant dans la plage allant de 10 : 1 à 4 : 1.

7. Formulation solide selon l'une quelconque ou plusieurs des revendications précédentes, la quantité de l'amidon alimentaires modifiés étant dans la plage allant de 10 à 50 % en poids, préférablement dans la plage allant de 25 à 45 % en poids, sur la base du poids total de la formulation.

8. Formulation solide selon l'une quelconque ou plusieurs des revendications précédentes, la quantité de sirop de glucose étant dans la plage allant de 0,1 à 40 % en poids, préférablement dans la plage allant de 10 à 30 % en poids, plus préférablement dans la plage allant de 15 à 25 % en poids, sur la base du poids total de la formulation.

9. Formulation solide selon l'une quelconque ou plusieurs des revendications précédentes, la quantité de l'antioxydant soluble dans l'eau (qui est préférablement l'ascorbate de sodium) étant dans la plage allant de 0,1 à 10 % en poids, préférablement dans la plage allant de 2 à 7 % en poids, plus préférablement dans la plage allant de 4 à 6 % en poids, sur la base du poids total de la formulation.

10. Formulation solide selon l'une quelconque ou plusieurs des revendications précédentes, la quantité du caroténoïde broyé, la quantité de l'au moins un amidon alimentaire modifié, la quantité du sirop de glucose, la quantité de saccharose et la quantité de l'antioxydant soluble dans l'eau (qui est préférablement l'ascorbate de sodium) totalisant une quantité d'au moins 90 % en poids, préférablement d'au moins 95 % en poids, sur la base du poids total de la formulation.

11. Formulation solide selon l'une quelconque ou plusieurs des revendications précédentes, la formulation ne contenant aucune huile.

12. Formulation solide selon l'une quelconque ou plusieurs des revendications précédentes, la formulation ne comprenant aucun des composés suivants : des produits de lécithine hydrolysée, une gomme arabique, des antioxydants solubles dans une graisse, l'isomalt, le α-zéacarotène et le β-zéacarotène.

13. Supplément diététique comprenant une formulation solide selon l'une quelconque ou plusieurs des revendications précédentes.

14. Supplément diététique selon la revendication 13 comprenant en outre des vitamines, des sels minéraux et/ou des oligoéléments.

15. Supplément diététique selon la revendication 13 et/ou 14, le supplément diététique étant sous la forme d'un comprimé.

16. Comprimé selon la revendication 15, le comprimé possédant une perte par compression de moins de 12 % de caroténoïde, sur la base de la quantité totale de caroténoïde.

17. Comprimé selon la revendication 15 et/ou 16, le comprimé étant un comprimé multivitaminémultiminéral présentant une dureté comme force d'écrasement d'au moins 70 N lorsqu'une force de compression de 10 kN est appliquée.

18. Utilisation d'une formulation solide selon l'une ou plusieurs des revendications 1 à 12 en tant qu'additif pour des suppléments diététiques, notamment pour des suppléments diététiques sous forme de comprimés.

19. Procédé pour l'obtention de la formulation solide selon la revendication 2, comprenant les étapes suivantes :
a) mise à disposition d'une solution aqueuse d'au moins un amidon alimentaire modifié, d'un sirop de glucose et de saccharose ;
b) ajout du caroténoïde à la solution de l'étape a) obtenant ainsi une suspension ;
c) broyage de la suspension de l'étape b) jusqu'à ce que la distribution de taille de particule du caroténoïde broyé selon la revendication 1 soit atteinte ;
d) granulation par pulvérisation de la suspension de l'étape c) pour obtenir la formulation solide selon l'une quelconque ou plusieurs des revendications 2 à 12 ;
un antioxydant soluble dans l'eau (qui est préférablement l'ascorbate de sodium) étant ajouté pendant le procédé.
